# EUROPÄISCHE PATENTSCHRIFT

(11) **EP 0 554 431 B1**
(45) Veröffentlichungstag und Bekanntmachung des Hinweises auf die Patenterteilung: **06.11.1996**
(21) Anmeldenummer: 92918000.8
(22) Anmeldetag: 28.08.1992
(51) Int. Cl.: G01N 11/14, G01N 33/38

(54) **GERÄT ZUM PRÜFEN VON FRISCHBETON UND MÖRTEL**
DEVICE FOR TESTING UNSET CONCRETE AND MORTAR
DISPOSITIF POUR LE CONTROLE DU BETON FRAICHEMENT MALAXE ET DU MORTIER

(30) Priorität: 28.08.1991 CH 2526/91
(43) Veröffentlichungstag der Anmeldung: 11.08.1993
(73) Patentinhaber: Atrof Bauphysik AG, CH-6300 Zug (CH); JENOPTIK Aktiengesellschaft, D-07743 Jena (DE)
(72) Erfinder: ENZLER, Ruedi, CH-9620 Lichtensteig (CH); LEMBKE, Elfrun, O-6902 Jena (DE); LÜTH, Gunter, O-6902 Jena (DE); ZIMMERMANN, Peter, O-6902 Jena (DE); SCHMIDT, Eberhard, O-6902 Jena (DE)
(74) Vertreter: Geyer, Werner, Dr.-Ing.
(86) Internationale Anmeldenummer: CH9200174
(87) Internationale Veröffentlichungsnummer: WO9305382

(56) Entgegenhaltungen:
- EP-A- 0 125 774
- DE-A- 3 237 090
- DE-U- 8 512 907
- FR-A- 2 165 178
- GB-A- 2 092 308
- US-A- 4 484 468
- PATENT ABSTRACTS OF JAPAN vol. 12, no. 436 (P-787)17. November 1988 & JP-A-63165756

## Beschreibung

Die vorliegende Erfindung betrifft ein Gerät zum Prüfen von Frischbeton und Mörtel.

Zur Durchführung vieler Prüfungem von Frischbeton und Mörtel werden an der Baustelle Proben entnommen und darauf in einem davon weit entfernten Labor geprüft. Da der Bau während diesen Prüfungen fortschreitet, sind Prüfungsergebnisse oft erst dann erhältlich, wenn der Bau schon viel weiter fortgeschritten ist, so dass Mängel erst dann erkannt werden, wenn eine Mängelbehebung praktisch nicht mehr möglich ist, z.B. wenn Teile des erstellten Bauwerks wieder abgebrochen werden müssten. Dieses ist eine sehr unbefriedigende Situation.

Weiter ist ein grosser Teil von an Betonkonstruktionen auftretenden Schäden auf Fehler bei der Verarbeitung des Betons zurückzuführen. Somit ist es notwendig, die von Unternehmen erzeugten Produkte an den Uebergabestellen zu prüfen, d.h. im Falle von Beton vor dem Giessen desselben. Beispielsweise sollten Prüfungen bei der Uebergabe des Frischbetons auf der Baustelle vom Transportbetonwerk an das bauausführende Unternehmen durchgeführt werden. Prüfungen von Frischbeton, auch für Festbeton und Ausgangsstoffe sind in der SIA-Norm 162, Ausgabe 1989 festgelegt. Um nun diese Prüfungen, insbesondere bei Frischbeton durchführen zu können, ist grundsätzlich eine grosse Anzahl unterschiedlichster Geräte notwendig gewesen. Ein derartiges Gerät ist aus GB-A-2 092 308 bekannt. Die Beschaffung und die Lagerung der vielen unterschiedlichen, von verschiedenen Herstellern zu beziehenden Geräte ist äusserst kostenaufwendig.

Ziel der Erfindung ist, die oben genannten Nachteile zu beheben.

Die erfindungsgemässe Vorrichtung ist gekennzeichnet durch eine Grundeinheit und eine Anzahl daran wahlweise anschliessbare Prüfgeräte zur Erzeugung von Messdaten, wobei mindestens ein Prüfgerät eine rotierbare Ausbildung aufweist, welche Grundeinheit mindestens einen Anschluss für ein jeweiliges Grundgerät, eine Energiequelle für einen netzunabhängigen Betrieb der Vorrichtung, eine Rechnungseinheit, eine Wahlvorrichtung zur Wahl der jeweils durchzuführenden Prüfung und damit der durch die Rechnungseinheit basiert auf den von den Prüfgeräten stammenden Messdateen durchzuführenden Rechnungsvorgänge, eine Anzeige und eine Wandlervorrichtung zum Umsetzen der von der Rechnungseinheit ausgegebenen Daten in mindestens bei der Anzeige lesbaren Prüfergebnisse aufweist.

Nachfolgend wird der Erfindungsgegenstand anhand der Zeichnungen beispielsweise näher erläutert.

Es zeigt:
Figur 1 eine erste Ausführung eines Prüfgerätes,
Figur 2 eine zweite Ausführung eines Prüfgerätes, welches mit dem Grundgerät verbunden ist, das seinerseits mit einer EDV-Anlage verbindbar ist,
Figur 3 eine weitere Ausführung eines Prüfgerätes,
Figur 4 eine Aufsicht auf einen sternförmigen Sondenkopf,
Figuren 5 und 6 als Rotationskörper ausgebildete Sondenköpfe von Prüfgeräten,
Figur 7 einen würfelförmig ausgebildeten Sondenkopf eines Prüfgerätes,
Figur 8 eine vereinfachte Seitenansicht einer weiteren Ausführung der erfindungsgemässen Vorrichtung mit einer weiteren Ausführung eines Prüfgerätes und Sondenkopfes,
Figur 9 schematisch einen Schnitt durch die in der Figur 8 gezeigte Grundeinheit,
Figur 10 eine Ansicht einer weiteren Variante von Sondenköpfen, und
Figur 11 ein Schaltschema.

Die gemäss der SIA-Norm (1968) vorgeschriebenen Prüfungen sind aus der nachfolgenden Tabelle ersichtlich.

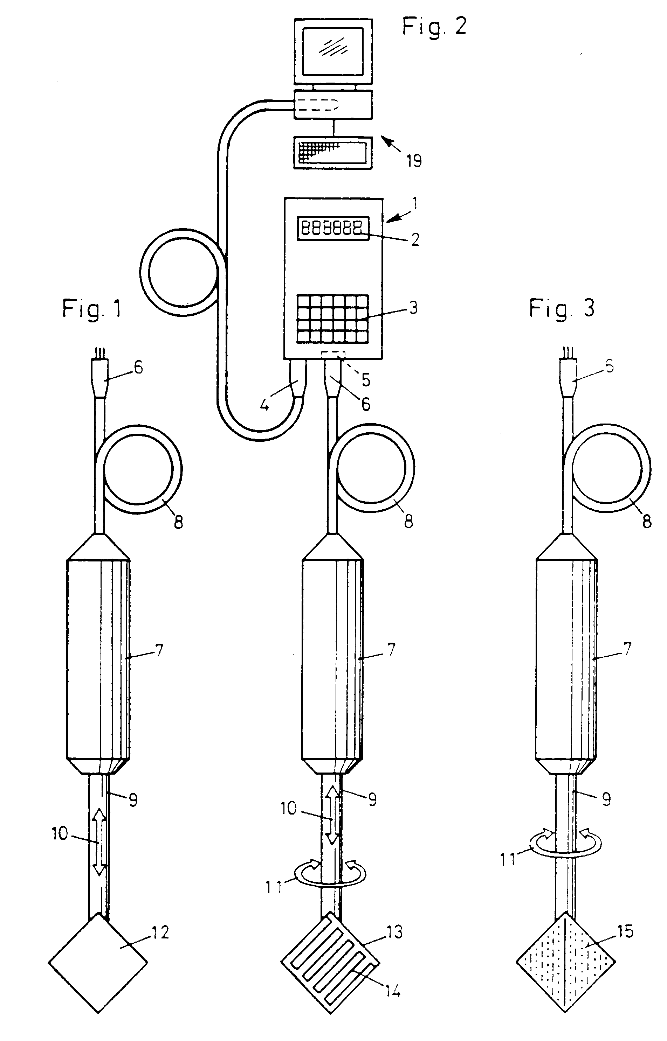

Die in den Figuren 1-3 gezeigte erste Ausführung der erfindungsgemässen Vorrichtung weist grundsätzlich eine tragbare Grundeinheit 1 auf. Die Abmessungen dieser Grundeinheit 1 entsprechen ungefähr denjenigen eines Taschenrechners. Die Grundeinheit 1 enthält eine Energiequelle in Form von einer oder mehreren Batterien, eine Anzeige 2, eine Rechnungseinheit auf die noch weiter unten eingegangen wird und eine Tastatur 3.

Weiter ist im Grundgerät 1 ein erster Speicher eingebaut, in welchem die ermittelten Messdaten gespeichert werden können. In einer weiteren Speichereinheit sind genormte Betonrezepturen in Form von Kennlinien gespeichert. Die Kennlinien sind insbesondere für eine vorgegebene Sieblinie, Herkunft der Zuschlagstoffe, Zusatzmittel (Frotschutz, Superverflüssier, Luftporenbildner, etc.) eingespeichert. Diese Kennlinien sind derart eingespeichert, dass die Normen der unterschiedlichsten Länder, in welchen die Vorrichtung zur Anwendung kommt, berücksichtigt sind. Ueber einen Anschluss 4 lässt sich die Grundeinheit 1 mit einer EDV-Anlage 19 verbunden, die beispielsweise für eine Archivierung, Statistik etc. dienen kann. Wie noch gezeigt wird, wird die Grundeinheit 1 mit den Prüfgeräten immer nur an der Baustelle verwendet. Die ermittelten Messdaten lassen sich in der Grundeinheit 1 speichern und nach Arbeitsende wird die Grundeinheit in z.B. das Büro des Bauingenieurs genommen und dort an die EDV-Anlage für die obengenannten Zwecke angeschlossen.

Die Grundeinheit lässt sich nun mit verschiedenen Prüfgeräten verbunden, die zur Durchführung der jeweiligen Prüfung dienen und die unterschiedliche Sondenköpfe enthalten können. Dazu weist die Grundeinheit 1 einen Eingangsanschluss 5 auf und die verschiedenen Prüfgeräte z.B. steckerförmige Anschlüsse, die beim Durchführen der jeweiligen Prüfung mit dem Eingangsanschluss 5 verbunden, d.h. eingesteckt werden.

Die Prüfgeräte weisen jeweils einen Handgriff 7 auf, siehe Figuren 1-3, wobei der jeweilige steckerförmige Anschluss 6 über ein Kabel 8 mit dem Handgriff 7 verbunden ist. Im jeweiligen Handgriff 7 ist eine elektronische Schaltung oder eine Steuerung entsprechend der jeweiligen Aufgabe angeordnet. Die in den Figuren 1-3 gezeichnete Form der Handgriffe 7 ist nicht zwingend, die können entsprechend der Aufgabe rund, quadratisch, lang oder kurz ausgebildet sein. Der Handgriff ist weiter über ein langgestrecktes Verbindungsglied 9 mit einem Messondenkopf verbunden. Das Verbindungsglied 9 kann starr oder biegsam sein, kann als Rohr- oder Schlauchleitung ausgebildet sein und weiter kann es elektrische Leitungen beinhalten, welche vom jeweiligen Sondenkopf zur elektronischen Schaltung in der Grundeinheit 1 kompatibel ist, bzw. entsprechend verarbeitet und angezeigt werden kann.

Im Falle eines starren Verbindungsgliedes kann entsprechend einer jeweiligen Aufgabe im Handgriff 7 ein Antrieb vorhanden sein, welcher, wie mit dem Doppelpfeil 10 in der Figur 1 angedeutet, eine vibrierende Hin- und Herbewegung des Sondenkopfes bewirken kann. Der Antrieb kann derart sein, dass er zusätzlich zur vibrierenden Bewegung gemäss dem Doppelpfeil 10 der Figur 2 eine Rotationsbewegung des Sondenkopfes bewirken kann, wie mit der Bezugsziffer 11 angedeutet ist und weiter ist es offensichtlich auch möglich den Antrieb so auszugestalten, dass er, wie in der Figur 3 gezeichnet ist, lediglich eine Rotationsbewegung gemäss der Bezugsziffer 11 des Sondenkopfes erzeugen kann.

Weiter können die Verbindungsglieder 9 rohrförmig oder schlauchförmig sein, so dass vom Handgriff 7 her gesteuert Druckwasser, Druckluft oder ein Vakuum (Saugzug) auf die Sondenköpfe übertragen werden kann.

Die Sondenköpfe können entsprechend der jeweiligen Aufgabe unterschiedliche Formen annehmen. Der in der Figur 1 gezeigte Sondenkopf 12 weist eine plattenförmige, in der Aufsicht quadratische Form auf. In diesem Sondenkopf 12 kann z.B. ein Temperaturfühler angeordnet sein, so dass die Ausführung nach der Figur 1 als Temperatursonde (ein Vibrieren ist hier nicht unbedingt notwendig) eingesetzt werden kann.

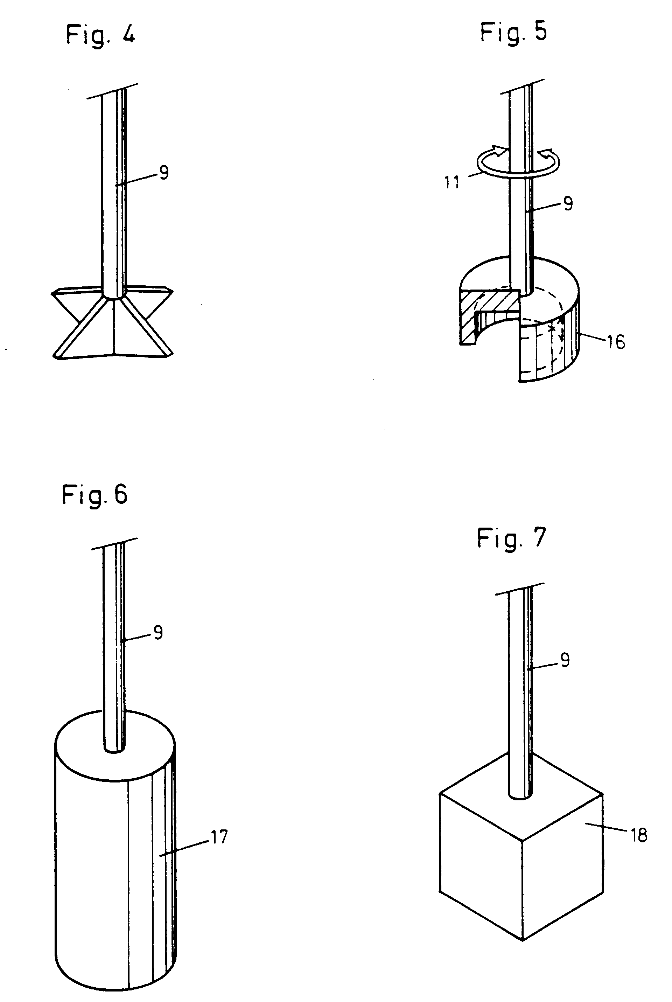

Der Sondenkopf 13 nach der Figur 2 weist wieder eine plattenförmige, in der Aufsicht quadratische Gestalt auf, wobei in derselben ein Leiter 14 eingesetzt ist. Diese Sonde kann z.B. (wieder nicht unbedingt beweglich) als Wasser/Zementwertsonde eingesetzt werden. Der mit Durchlöcherungen 15 ausgerüstete Sondenkopf) nach Figur 3 kann als Konsistenzsonde eingesetzt werden, wobei diese wie in der Figur 4 gezeigt, in der Aufsicht sternförmig sein kann. Ein ringförmiger Sondenkopf 16 ist in der Figur 5 gezeigt. Dieser lässt sich ebenfalls in Rotation versetzen um beispielsweise einen Abrieb zu erzeugen, wobei der Abrieb beispielsweise durch das rohrförmige Verbindungsglied 9 in den Handgriff 7 zur Analyse eingesogen werden kann. Ein walzenförmiger Sondenkopf 17 ist in der Figur 6 gezeigt. Die Figur 7 zeigt einen würfelförmigen Sondenkopf 18. Obwohl hier das Verbindungsglied 9 von einer Seite des Würfels 18 absteht, kann das Verbindungsglied 9 auch derart angebracht sein, dass es mit einer Raumdiagonale des Würfels 18 fluchtet.

Die Abmessungen der Sondenköpfe können, betrachtet man die Seitenlängen, einige Zentimeter bis einen Dezimeter je nach auszuführender Aufgabe und in denselben vorhandenen Sondeneinrichtungen betragen.

Je nach der durchzuführenden Prüfung z.B. SIA 162 (1968) wird nun ein Sondenkopf ausgewählt und über den steckerförmigen Anschluss 6 mit der Grundeinheit 1 verbunden. Mittels der Tastatur 3 wird nun in der Grundeinheit ausgewählt, welche Rechnungsvorgänge in der Rechnungseinheit durchgeführt werden, um eine jeweilige digitale Anzeige im Feld 2 zu erhalten. Diese Anzeige kann noch durch eine ebenfalls erscheinende Identifikation der jeweiligen Prüfung ergänzt werden, so dass, wie in der Figur 2 gezeigt, beim Durchführen der Wasser/Zementwertprüfung nicht nur die entsprechende digitale Anzeige sondern noch identifizierende Buchstaben, hier W/Z angezeigt werden. Weitere Tasten dienen zum Beginnen bzw. Stoppen der Messung, zum Speichern der Messwerte und offensichtlich zum Uebertragen derselben auf die EDV-Anlage.

Die Rechnungseinheit in der Grundeinheit 1 ist weiter derart ausgebildet, dass sie aus mehreren Messwerten einen weiteren Wert entsprechend einer durchzuführenden Prüfung errechnen und anzeigen kann.

Nachfolgend sind nun Beispiele der mit der erfindungsgemässen Vorrichtung durchführbaren Messungen bzw. Prüfungen aufgelistet, wobei die Numerierung derjenigen der Prüfungsnummern der obigen SIA-Tabelle entsprechen.
- Nr. 1/2: Zylinderdruckbehälter
- Nr. 3: elektronisch errechnter E-Modul 1/2 und 4
- Nr. 4: Laserdeformationsmessung auf der Basis der holographischen Interferometrie
- Nr. 5: errechnete Wasserleitfähigkeit aus 1/2, 3, 4
- Nr. 7: errechnete Wasserleitfähigkeit aus 1/2, 3, 4, 5
- Nr. 8: Hohlkörpermessung durch Temperaturwechselbehälter sowie errechnete Werte Nr. 5
- Nr. 9: Hohlkörpermessung durch Temperaturwechselbehälter sowie errechnete Werte Nr. 5
- Nr. 10: Rotationsbehälter mit Abriebsondenoberflächen
- Nr. 12: chemische Zusatzsonde mit integriertem Chemikalienbehälter
- Nr. 13: elektronisch errechneter Wert aus 1/2, 3, 19, 18
- Nr. 16: wie Nr. 12
- Nr. 17: wie Nr. 12
- Nr. 18: errechnete Werte aus Nr. 19 + 20
- Nr. 19: Feuchtigkeitssonde im Verhältnis der vorgegebenen Zementdosierung und Nr. 20
- Nr. 20: Rotationssonde mit Oberflächenprofil (stern, walzen, geriffelt) je nach max. Betonkorndurchmesser (Steine)

Um die Würfel- bzw. Bohrkerndruckfestigkeit, also Prüfung Nr. 1 oder 2 zu durchzuführen, wird als Sondenkopf ein Zylinderdruckbehälter z.B. gemäss der Figur 6 verwendet, wobei noch festgehalten werden soll, dass dessen Seitenwand nicht starr, sondern auch flexibel ausgebildet sein kann. Der Kriech- und Schwindwert, Prüfung Nr. 4, wird mittels einer Laserdeformationsmessung auf der Basis der holographischen Interferometrie durchgeführt. Nachdem die Prüfungen gemäss den Nummern 1, 2 und 4 durchgeführt und deren Werte in der Grundeinheit 1 gespeichert worden sind, lässt sich aus diesen drei Prüfungen der E-Modul elektronisch in der Grundeinheit 1 errechnen, wozu wieder ein einfacher entsprechender Tastendruck notwendig ist.

SIA-Prüfung Nr. 5, Wasserleitfähigkeit, wird aus den Werten der Prüfungen Nr. 1/2, 3 und 4 errechnet. Die Porosität, Nr. 7, ergibt sich errechnet aus den Daten der Prüfungen Nr. 1/2, 3 und 5. Prüfung Nr. 8, Frostwechselverhalten, ergibt sich durch eine Hohlkörpermessung durch einen Temperaturwechselbehälter, wobei der Wert Frostwechselverhalten sich aus dieser Messung sowie der errechneten Werte nach Prüfung Nr. 5 ergeben. Das Frost-Tausalzverhalten, Prüfung Nr. 9, ergibt sich wieder durch eine Hohlkörpermessung durch einen Temperaturwechselbehälter sowie der errechneten Werte nach Prüfung Nr. 5.

Das Abriebverhalten, Prüfung Nr. 10, wird mit einem Rotationsbehälter mit Abriebsondenoberflächen durchgeführt, also einen Sondenkopf, der etwa gemäss dem Sondenkopf 16 nach Figur 5 ausgebildet ist. Die Sauberkeit, Prüfung Nr. 12, wird mit einer chemischen Zusatzsonde mit integriertem Chemikalienbehälter, etwa in der Form des Sondenkopfes 17 nach Figur 6 oder auch Sondenkopfes 18 nach Figur 7 durchgeführt. Der Mehlkorngehalt, Prüfung Nr. 13 ergibt sich aus dem elektronisch errechneten Wert aus Prüfungen Nr. 1/2, 3, 10 und 18 (siehe weiter unten). Die Prüfung des Anmachwassers, Prüfung Nr. 16 sowie die Prüfung der Eignung (Zusatzmittel und Zusatzstoffe), Prüfung Nr. 17, werden mit einer chemischen Zusatzsonde, wie dies der Fall bei der Prüfung Nr. 12 ist, durchgeführt. Die Ergiebigkeit, Prüfung Nr. 18, ergibt sich aus einem errechneten Wert, der aus den Prüfungen Nr. 19 und 20 errechnet wird.

Der Wassergehalt und Wasserzementwert, Prüfung Nr. 19, wird mit einer Feuchtigkeitssonde (siehe auch Figur 2) im Verhältnis der vorgegebenen Zementdosierung und aus den Werten nach Figur 20 ermittelt.

Die Konsistenzprüfung, Prüfung Nr. 20, wird je nach dem maximalen Betonkorndurchmesser (Steine) mit einer Rotationssonde mit einem Oberflächenprofil des Sondenkopfes z.B. Figur 5 oder auch Figur 6 durchgeführt, wobei dieser walzenförmig sein kann, geriffelt sein kann oder auch mit einem Sondenkopf, der in der Aufsicht gemäss der Abbildung nach Figur 4 ausgebildet, also sternförmig ist.

Es ist also ersichtlich, dass je nach der durchzuführenden Prüfung die dazu ausgebildete Sonde, bzw. Sondenkopf ausgewählt und das entsprechende Prüfgerät an die Grundeinheit angeschlossen wird und darauf durch Drücken der entsprechenden Taste 3 über die Rechnungseinheit in der Grundeinheit die Anzeige 2 aktiviert wird. Wenn eine Anzeige aus mehreren ermittelten Werten durchzuführen ist, werden die entsprechenden Prüfungen vorgängig wieder mittels der entsprechenden Prüfgeräte durchgeführt, wobei die Werte im Speicher der Grundeinheit 1 gespeichert werden. Durch ein Drücken der entsprechenden Taste wird dann ein auf diesen gespeicherten Werten basiertender Rechnungsvorgang durchgeführt und dann wieder in der Anzeige 2 das Ergebnis angezeigt.

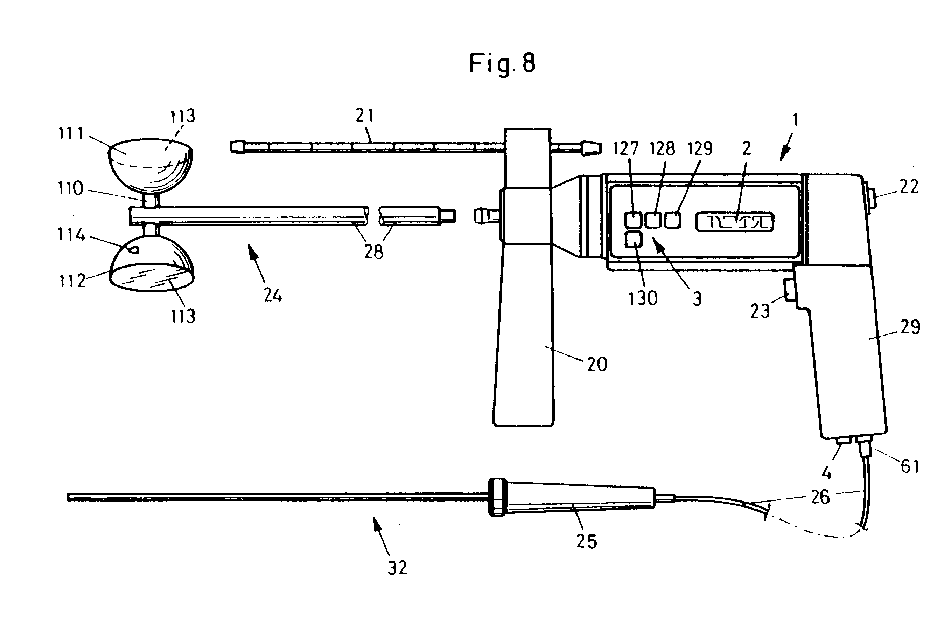

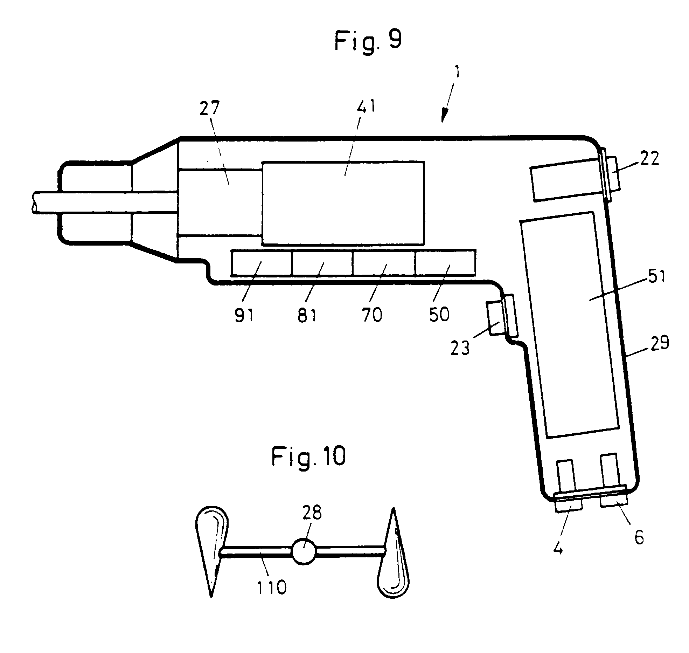

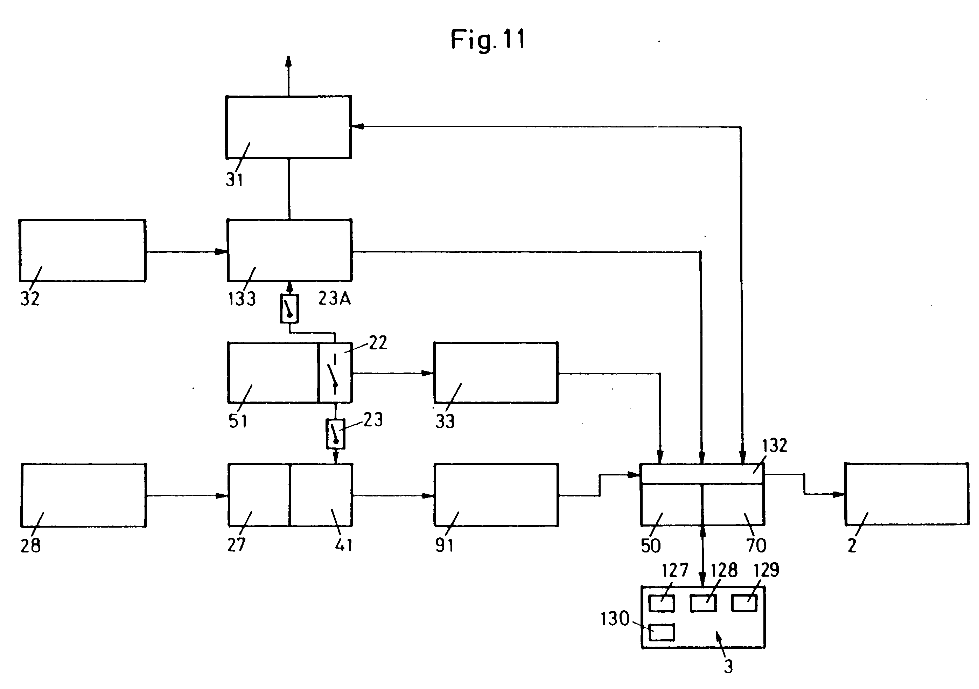

Eine weitere Ausführung der erfindungsgemässen Vorrichtung ist in den Figuren 8 und 9 gezeigt. Das dazugehörige Blockschema ist in der Figur 11 dargestellt, wobei festzuhalten ist, dass der Grundaufbau dieses Blockschemas auch im Schaltkreis der oben erläuterten, ersten Ausführung der erfindungsgemässen Vorrichtung ausgeführt ist.

Diese weitere Ausführung enthält wieder die Grundeinheit 1, in welcher ein Motor 41 und ein Getriebe 27 angeordnet sind. Weiter sind in der Grundeinheit 1 elektronische Schaltkreise vorhanden, nämlich eine Speichereinheit 70 für Kennlinien, eine Wahlvorrichtung 81, eine Wandlervorrichtung 91 und einen Speicher 50 für die Messdaten. Die Funktion dieser elektronischen Schaltungen (die für beide Ausführungen zutreffend sind) wird weiter unten erläutert werden. Im Pistolengriff 29 ist ein nachladbarer Akkumulator 51 angeordnet. An diesem ist über die Steckverbindung 61 ein Ladegerät anschliessbar. Aufgrund des Akkumulators 51 ist das Gerät netzunabhängig einsetzbar. Das heisst, zum Durchführen der verschiedenen Prüfungen muss bei der jeweiligen Prüfstelle kein Anschluss an ein elektrisches Netz vorhanden sein.

Die Grundeinheit 1 weist einen weiteren Handgriff 20 auf. Um sicherzustellen, dass eine jeweilige Folge von Messungen immer in derselben Tiefe im Frischbeton durchgeführt werden, ist im Handgriff 20 ein Messstab 21 eingesetzt, der im Handgriff 20 längsverschiebbar angeordnet ist, so dass die jeweilige Eintauchtiefe der Messonden, bzw. Sondenköpfe genau gemessen werden kann.

Weiter weist die Grundeinheit 1 einen Ein/ Aus-Schalter 22 auf. Mittels diesem Schalter 22 wird grundsätzlich die Stromzufuhr zu den verschiedenen elektrischen und elektronischen Einheiten in der Grundeinheit eingeschaltet bzw. ausgeschaltet.

Die einzelnen Messungen werden (bei eingeschaltetem Schalter 22) durch den Schalter 23 ausgelöst. Beide Schalter 22, 23 sind, wie aus der Zeichnung ersichtlich ist Druckknopfschalter. Bezugnehmend auf Figur 11 ist im Schaltkreis unmittelbar nach dem Schalter 22 eine Ueberwachungseinheit 33 einschliesslich einer Ueberlastsicherung angeordnet. Dieser nachfolgend ist dann der Mikrokontroller 132 mit den Speichern 50 und 70 angeordnet. Die durch das Einschalten des Schalters 22 vom Akkumulator 51 her kommende elektrische Energie wird, um Beschädigungen des Schaltkreises zu vermeiden, durch die Einheit 33 überwacht. Neben der Steckverbindung 61 ist ein weiterer Anschluss 4 vorhanden. Dieser dient wieder zur Verbindung des Gerätes mit einer elektronischen Datenverarbeitungsanlage (siehe Fig. 2). Dabei können im Speicher 50 gespeicherte Messdaten einem elektronischen Rechner zur weiteren Auswertung übertragen werden. Die dazugehörige PC-Schnittstelle ist in der Figur 11 mit der Bezugsziffer 31 bezeichnet.

An die Grundeinheit 1 lassen sich nun wieder verschiedene Prüfgeräte anschliessen. Ein erstes beispielsweises Prüfgerät 24 ist die sogenannte Konsistenzsonde. Das Prüfgerät weist einen Tragstab 28 auf, der mit dem Getriebe 27 über eine Schraubverbindung verbunden und entsprechend durch den Motor 41 rotiert werden kann. Der Tragstab 28 weist beim entfernten Ende ein Querstück 110 auf. Dieses Querstück 110 trägt zwei halbkugelförmige Sondenköpfe 111 und 112. Die Bezugsziffer 113 bezeichnet den ebenen Oberflächenabschnitt der Halbkugel des Sondenkopfes 111. In einem oder beiden Sondenköpfen 111, 112 lassen sich zusätzlich weitere Messonden einsetzen. Als Beispiel ist im Sondenkopf mit der Bezugsziffer 114 die Einsatzstelle für eine Feuchtigkeitssonde angedeutet. Bei eingeschaltetem Schalter 22 und nachfolgendem Eindrücken des Schalters 23 lässt sich somit das Prüfgerät 24 zur Durchführung der Prüfungen rotieren.

Die Bezugsziffer 32 bezeichnet eine Temperaturprüfgerät mit Handgriff 25. Das Temperaturprüfgerät 32 ist über ein Kabel 26 an die Steckverbindung 61 anschliessbar. Die Steckverbindung 61 dient also einerseits zum Anschluss an ein Ladegerät für den Akkumulator und andererseits als Anschluss für den Temperaturfühler, d.h. Prüfgerät 32.

Seitlich an der Grundeinheit 1 ist die Anzeige 2 vorhanden, in welchem verschiedene Anzeigen, auf die weitere unten noch eingegangen wird, erscheinen. Weiter ist an der Grundeinheit 1 eine Folientastatur angeordnet. Dabei ist die Taste 127 die Temperaturwähltaste, d.h. bei Drücken dieser Taste 127 lässt sich in der Grundeinheit 1 bei angeschlossenem Temperaturprüfgerät 32 eine Temperaturmessung durchführen, wobei die Temperatur im Feld der Anzeige 2 angezeigt wird.

Die Taste 128 dient zur Auswahl verschiedener Messdaten, die bei rotierender Messonde 28 ermittelt werden, wobei die Taste 128 zur Auswahl einer jeweils durchzuführenden Messung aufeinanderfolgend gedrückt werden muss. Die Taste 129 dient zum Auswählen einer der in der Speichereinheit 70 eingespeicherten Kennlinie für die zu untersuchende Frischbetonrezeptur und die Taste 130 dient schliesslich zum Kalibrieren der Vorrichtung.

Das rotierende Prüfgerät 28 ist grundsätzlich eine Konsistenzsonde. Sie ist vom Motor 41 über das Getriebe 27 mit einer Drehzahl von ungefähr 5-20 U/min. angetrieben.

Das Querstück 110 mit den zwei damit fest verbundenen Sondenköpfen 111 und 112 ist um seine Längsaxe drehbar mit dem Tragstab 11 verbunden. Damit kann die Winkelstellung der halbkugelförmigen Sondenköpfe 111, 112 entsprechend der Konsistenz der Masse, in welcher sie eingetaucht wird, frei gewählt werden. Es kann z.B. eine beliebige Schiefstellung der ebenflächigen Oberflächenabschnitte 113 relativ zum Tragstab 28 gewählt werden. Es ist auch eine 180°-Aenderung der Drehstellung der Sondenköpfe 111, 112 um das Querstück 110 möglich. Bei Beton ist die Stellung der Sondenköpfe 111, 112 beispielsweise derart, dass der jeweils gewölbte Oberflächenabschnitt der halbkugelförmigen Sondenköpfe 111, 112 vorläuft. Im Falle von Mörtel, der eher dünnflüssig ist, ist eher der ebenflächige Oberflächenabschnitt 113 der halbkugelförmigen Sondenköpfe 111, 112 vorlaufend.

Wie in der Aufsicht nach Figur 10 gezeigt ist, können auch andere Ausführungen der Sondenköpfe zur Anwendung kommen. Beispielsweise lassen sich auch tropfenförmige Sondenköpfe gemäss der in Figur 10 gezeichneten Ausführung verwenden.

Im Betrieb rotiert das Prüfgerät, wobei in Abhängigkeit der aufzuwendenden Kraft (gemessen durch die vom Motor aufgenommene Leistung) und des Hebelarmes der Sonde über in der Wandlervorrichtung 91 (siehe Fig. 11) ein Drehmoment ermittelt werden kann, dessen Ausgang über den Mikrokontroller 132 dem Anzeigefeld 2 zugeführt ist, in welchem der skalare Wert (sog. FCT-Wert) angezeigt wird.

In der Speichereinheit 50 sind genormte Betonrezepturen in Form von Kennlinien gespeichert. Die Kennlinien sind insbesondere für eine vorgegebene Sieblinie, Herkunft der Zuschlagstoffe, Zusatzmittel (Frostschutz, Superverflüssiger, Luftporenbildner, etc.) eingespeichert, wobei auch die Normen verschiedener Länder in entsprechender Kennlinien berücksichtigt sind.

Der Messvorgang erfolgt folgendermassen. Vorerst wird mittels des Messstabes 21 die erwünschte Eintauchtiefe der Sondenköpfe 111, 112 festgelegt und danach durch Drücken des Ein/Aus-Schalters 22 die Vorrichtung eingeschaltet. Danach wird die Taste 23 gedrückt; der Tragstab 28 mit den Sondenköpfen 111, 112 rotiert in der Luft und es erfolgt die interne Kalibrierung des Gerätes. Dieser Vorgang dauert ungefähr 4 Sekunden und endet automatisch. Danach werden die Messonden 111, 112 in z.B. den Frischbeton gesteckt, bis der Messstab 21 die Betonoberfläche berührt. Durch Drücken des Schalters 23 wird das Prüfgerät in Betrieb gesetzt, die zum Rotieren notwendige Kraft nach bekannten Vorgängen gemessen und über die Wandlervorrichtung 91 und dem Mikrokontroller 132 im Anzeigefeld 26 als sog. FCT-Anzeige angegeben. Auch hier wird die Zeitdauer des Rotierens automatisch beendet. Danach kann dieselbe Messung, dasselbe Vorgehen an verschiedenen Stellen des zu prüfenden Betons durchgeführt werden. Aus den verschiedenen Messungen ergibt sich ein Mittelwert. Dieser wird im Speicher 50 gespeichert. Das Prüfgerät 24 wird aus dem Beton herausgehoben.

Für die Ausgabe von Messwerten wird folgendermassen vorgegangen. Durch Druck auf die Taste 129 wird die Speichereinheit 50 angesteuert, so dass im Anzeigefeld 2 Angaben wie Sieblinie, Grösstkorn, Nummer der eingespeicherten Kennlinie für eine vorgegebene Betonrezeptur erscheinen. Ein notwendigerfalls wiederholtes Drücken der Taste 129 lässt dann diese Angaben für den zu messenden Beton erscheinen.

Durch ein erstes Drücken der Taste 128 wird, basiert auf den durch den Betrieb des Gerätes 24 ermittelten Wert, das Ausbreitmass A des Betons angezeigt. Der angezeigte Wert ist auf die in der Speichereinheit 70 gespeicherte und mittels der Messtaste 128 und Wahlvorrichtung 81 abgerufene Kennlinie basiert.

Ein nachfolgendes Drücken der Taste 128 lässt den Wasser-Zement-Wert abrufen und in der Anzeige 2 anzeigen. Der angegebene Wasser-Zement-Wert ist wieder auf der in der Speichereinheit 70 gespeicherten und durch die Wahlvorrichtung 81 gewählte Kennlinie basiert. Ein drittes Drücken der Taste 128 lässt die Druckfestigkeit D nach 28 Tagen anzeigen.

Diese drei Messdaten können unmittelbar abgelesen und beurteilt werden. Es ist nun auch möglich durch eine anschliessende Betätigung der Kalibriertaste 130 die angezeigten Werte im Speicher 50 zu speichern. Diese im Speicher 50 gespeicherten Messdaten können dann beispielsweise in einem Labor auf einen PC überspielt werden.

Zur Temperaturmessung dient das Prüfgerät 32. Dieses wird über die Steckverbindung 61 mit der Grundeinheit 1 verbunden. Durch Einschalten mittels dem Schalter 22 wird die Vorrichtung in Betrieb gesetzt. Danach wird die Taste 127 gedrückt, womit im Anzeigefeld 2 die vom Temperaturfühler gemessene Temperatur angezeigt wird, wobei ein gemittelter Wert mehrerer Werte angegeben werden kann.

Der Ausgang des Prüfgerätes 32 ist einem Temperaturmessmodul 133 zugeführt, in welchem der vom Prüfgerät 32 gelieferte Messwert in ein Signal zur Uebermittlung an den Mikrokontroller 132 umgesetzt wird, von welchem schliesslich das für das Anzeigefeld 2 notwendige Signal demselben geliefert wird. Der Momentanwert der Temperatur kann durch ein nochmaliges Drücken des Schalters 23 zur Anzeige gebracht werden, wobei der Schalter 23A der Fig. 10 geschlossen wird.

Somit lässt sich mit einer einfachen Vorrichtung beispielsweise gemäss den gezeigten Ausführungen der erfindungsgemässen Vorrichtung Frischbeton, Mörtel und ähnliche zementgebundene Massen vor Ort prüfen, so dass etwelche Mängel unmittelbar festgestellt werden können.

## Patentansprüche

1. Vorrichtung zum Prüfen von Frischbeton und Mörtel, gekennzeichnet durch eine Grundeinheit (1) und eine Anzahl daran wahlweise anschliessbare Prüfgeräte (28, 32) zur Erzeugung von Messdaten, wobei mindestens ein Prüfgerät eine rotierbare Ausbildung (11) aufweist, welche Grundeinheit mindestens einen Anschluss (6) für ein jeweiliges Prüfgerät, eine Energiequelle (5) für einen netzunabhängigen Betrieb der Vorrichtung, eine Rechnungseinheit, eine Wahlvorrichtung (81) zur Wahl der jeweils durchzuführenden Prüfung und damit zur Wahl der durch die Rechnungseinheit, basierend auf den von den Prüfgeräten stammenden Messdaten, durchzuführenden Rechnungsvorgänge, eine Anzeige (2) und eine Wandlervorrichtung (91) zum Umsetzen der von der Rechnungseinheit ausgegebenen Daten in mindestens bei der Anzeige lesbare Prüfergebnisse aufweist.

2. Vorrichtung nach Anspruch 1, dadurch gekennzeichnet, dass die Grundeinheit (1) einen Speicher (50) zum Speichern der Messdaten und einen Anschluss (4) zur Uebertragung der gespeicherten Messdaten auf eine EDV-Anlage (19) aufweist.

3. Vorrichtung nach Anspruch 1 oder 2, dadurch gekennzeichnet, dass die Grundeinheit (1) eine Speichereinheit (70) zum Speichern genormter Betonrezepturen aufweist, die durch die Wahlvorrichtung (81) abrufbar und der Wandlervorrichtung zuführbar ist.

4. Vorrichtung nach einem der Ansprüche 1-3, gekennzeichnet durch ein stationäres Prüfgerät, das mit einem Temperaturfühler (32) ausgerüstet ist.

5. Vorrichtung nach einem der Ansprüche 1-4, dadurch gekennzeichnet, dass mindestens eine Anzahl Prüfgeräte einen Sondenkopf (112) aufweisen, in welchem eine zusätzliche Messonde (114) eingebaut ist.

6. Vorrichtung nach einem der Ansprüche 1-5, gekennzeichnet durch ein Prüfgerät, das einen plattenförmigen, in der Aufsicht quadratischen Sondenkopf (12) aufweist.

7. Vorrichtung nach einem der Ansprüche 1-5, gekennzeichnet durch ein Prüfgerät, das einen würfelförmigen Sondenkopf (18) aufweist.

8. Vorrichtung nach einem der Ansprüche 1-5, gekennzeichnet durch ein Prüfgerät, das einen sternförmigen Sondenkopf aufweist.

9. Vorrichtung nach einem der Ansprüche 1-5, gekennzeichnet durch ein Prüfgerät, das einen ring- oder walzenförmigen Sondenkopf (16, 17) aufweist.

10. Vorrichtung nach einem der Ansprüche 1-9, gekennzeichnet durch mindestens ein Prüfgerät mit einem Sondenkopf (111, 112) und einem Handgriffabschnitt (29), in welchem Handgriffabschnitt mindestens eine elektronische Schaltung (23) oder Steuerung eingebaut ist, welche dazu dient, durch den Betrieb des Sondenkopfes entstehende Messwerte zur Weitergabe an die Rechnungseinheit umzuwandeln, welcher Sondenkopf über ein langgestrecktes, starres oder biegsames Verbindungsglied (9) mit dem Handgriffabschnitt verbunden ist.

11. Vorrichtung nach Anspruch 10, bei welcher der Sondenkopf über ein starres Verbindungsglied mit dem Handgriffabschnitt verbunden ist, dadurch gekennzeichnet, dass das Verbindungsglied mit einem im Handgriffabschnitt (20) angeordneten Antrieb verbunden ist, der dazu dient, eine Rotationsbewegung und/oder eine vibrierende Hin- und Herbewegung des Verbindungsgliedes (9) zu erzeugen.

12. Vorrichtung nach Anspruch 10, dadurch gekennzeichnet, dass das Verbindungsglied (9) als Rohr- oder Schlauchleitung zur Uebertragung von Druckwasser, Druckluft oder Vakuum ausgebildet ist.

13. Vorrichtung nach einem der Ansprüche 1-5, gekennzeichnet durch mindestens ein rotierbares Prüfgerät mit einem Sondenkopf und einem starren, langgestreckten Verbindungsglied, durch einen in der Grundeinheit angeordneten Antrieb einschliesslich einem Motor (41), wobei das starre Verbindungsstück (9) zur lösbaren Verbindung mit dem Antrieb ausgebildet ist.

14. Vorrichtung nach Anspruch 13, dadurch gekennzeichnet, dass das rotierbare Prüfgerät einen an den Antrieb anschliessbaren Tragstab (28) aufweist, der an einem Ende zwei von einem Querstück (110) einander diametral gegenüber angeordnet getragene Sondenköpfe (111, 112) aufweist.

15. Vorrichtung nach Anspruch 14, dadurch gekennzeichnet, dass die Sondenköpfe (111, 112) halbkugelförmig oder tropfenförmig ausgebildet und starr mit dem Querstück (110) verbunden sind, dass das Querstück (110) ein langgestrecktes Stangenglied ist, das zusammen mit den Sondenköpfen (111, 112) um seine Längsachse drehbar in unterschiedlichen Drehstellungen mit dem Tragstab (28) verbindbar ist, so dass die Drehstellung der Sondenköpfe um die Längsachse des Stangengliedes frei wählbar ist.

## Claims

1. Device for testing unset concrete and mortar, characterised by a basic unit (1) and, attachable thereto as desired, a number of test apparatuses (28, 32) for producing measurement data, at least one test apparatus comprising a rotatable construction (11), which basic unit comprises at least one connection (6) for a respective test apparatus, an energy source (5) for a mains-independent operation of the device, a calculator unit, a selector (81) for selecting the particular test to be carried out and thus for selecting the calculation processes to be carried out by the calculator unit based on the measurement data coming from the test apparatuses, a display (2) and a transducer (91) for converting the data delivered by the calculator unit into test results readable at least on display.

2. A device according to claim 1, characterised in that the basic unit (1) comprises a memory (50) for storing the measurement data and a connection (4) for transmitting the stored measurement data to EDP equipment (19).

3. A device according to claim 1 or 2, characterised in that the basic unit (1) comprises a memory unit (70) for storing standard concrete formulations, which is arranged to be fetched by the selector (81) and supplied to the transducer.

4. A device according to one of claims 1 to 3, characterised by a stationary test apparatus which is equipped with a temperature sensor (32).

5. A device according to one of claims 1 to 4, characterised in that at least a number of test apparatuses have a probe head (112) with a built-in additional measurement probe (114).

6. A device according to one of claims 1 to 5, characterised by a test apparatus which comprises a plate-like probe head (12) that is square in plan view.

7. A device according to one of claims 1 to 5, characterised by a test apparatus which comprises a cubic probe head (18).

8. A device according to one of claims 1 to 5, characterised by a test apparatus which comprises a star-shaped probe head.

9. A device according to one of claims 1 to 5, characterised by a test apparatus which comprises an annular or roller-shaped probe head (16, 17).

10. A device according to one of claims 1 to 9, characterised by at least one test apparatus having a probe head (111, 112) and a hand grip portion (29) in which there is incorporated at least one electronic circuit (23) or control means which serves to convert measured values resulting from operation of the probe head for relay to the calculator unit, the probe head being connected by way of an elongate rigid or flexible connecting member (9) to the hand grip portion.

11. A device according to claim 10, in which the probe head is connected by way of a rigid connecting member to the hand grip portion, characterised in that the connecting member is connected to a drive means arranged in the hand grip portion (20), which drive means serves to generate a rotary movement and/or a vibrating back and forth movement of the connecting member (9).

12. A device according to claim 10, characterised in that the connecting member (9) is in the form of a tube or hose line for transmission of water under pressure, compressed air or a vacuum.

13. A device according to one of claims 1 to 5, characterised by at least one rotatable test apparatus having a probe head and a rigid, elongate connecting member, by a drive means including a motor (41) arranged in the basic unit, the rigid connecting member (9) being designed for releasable connection to the drive means.

14. A device according to claim 13, characterised in that the rotatable test apparatus includes a supporting rod (28) attachable to the drive means, which supporting rod at one end has two probe heads (111, 112) carried by a cross member (110) and arranged diametrically opposite one another.

15. A device according to claim 14, characterised in that the probe heads (111, 112) are of semi-spherical or tear drop-shaped construction and are rigidly connected to the cross member (110), the cross member (110) is an elongate rod member which together with the probe heads (111, 112) is arranged to be connected, so that it is rotatable about its longitudinal axis, in different rotated positions to the supporting rod (28), so that the rotated position of the probe heads about the longitudinal axis of the rod member is freely selectable.

## Revendications

1. Dispositif d'essai de béton frais et de mortier, caractérisé par une unité de base (1) et un nombre d'appareils d'essai (28, 32), pouvant être raccordés au choix, pour la production de données de mesure, dans lequel un appareil d'essai au moins présente une formation (11) rotative, l'unité de base comportant au moins un raccord (6) pour un appareil d'essai respectif, une source d'énergie (5) pour un fonctionnement du dispositif, indépendant du réseau, une unité de calcul, un dispositif de sélection (81) pour la sélection de l'essai à réaliser et donc pour la sélection des opérations de calcul à effectuer par l'unité de calcul, sur la base des données de mesure provenant des appareils d'essai, un afficheur (2) et un dispositif à convertisseur (91) pour la conversion des données, délivrées par l'unité de calcul, dans au moins des résultats d'essai lisibles dans l'afficheur.

2. Dispositif selon la revendication 1, caractérisé en ce que l'unité de base (1) comporte une mémoire (50) pour la mémorisation des données de mesure et un raccord (4) pour le transfert des données de mesure mémorisées à un système informatique (19).

3. Dispositif selon la revendication 1 ou 2, caractérisé en ce que l'unité de base (1) comporte une unité à mémoire (70) pour la mémorisation de recettes de béton normalisées, qui peuvent être appelées par le dispositif de sélection (81) et envoyées au dispositif à convertisseur.

4. Dispositif selon l'une des revendications 1 à 3, caractérisé par un appareil d'essai fixe, qui est équipé d'une sonde de température (32).

5. Dispositif selon l'une des revendications 1 à 4, caractérisé en ce qu'au moins un nombre d'appareils d'essai comporte une tête de sonde (112), dans laquelle est intégrée une sonde de mesure (114) supplémentaire.

6. Dispositif selon l'une des revendications 1 à 5, caractérisé par un appareil d'essai, qui comporte une tête de sonde (12) en forme de plaque, carrée, vue de dessus.

7. Dispositif selon l'une des revendications 1 à 5, caractérisé par un appareil d'essai, qui comporte une tête de sonde (18) en forme de cube.

8. Dispositif selon l'une des revendications 1 à 5, caractérisé par un appareil d'essai, qui comporte une tête de sonde en forme d'étoile.

9. Dispositif selon l'une des revendications 1 à 5, caractérisé par un appareil d'essai, qui comporte une tête de sonde (16, 17) en forme d'anneau ou de rouleau.

10. Dispositif selon l'une des revendications 1 à 9, caractérisé par au moins un appareil d'essai avec une tête de sonde (111, 112) et une portion de poignée (29), dans cette portion de poignée étant intégré au moins un circuit électronique (23) ou une commande, qui sert à convertir les valeurs de mesure, résultant du fonctionnement de la tête de sonde, en vue de leur transmission à l'unité de calcul, laquelle tête de sonde est reliée à la portion de poignée, par un organe de liaison (9) allongé, rigide ou flexible.

11. Dispositif selon la revendication 10, dans lequel la tête de sonde est reliée à la portion de poignée, par un organe de liaison rigide, caractérisé en ce que l'organe de liaison est relié à un entraînement, placé dans la portion de poignée (20), qui sert à produire un mouvement de rotation et/ou un mouvement vibrant de va-et-vient de l'organe de liaison (9).

12. Dispositif selon la revendication 10, caractérisé en ce que l'organe de liaison (9) est conformé en conduite de tube ou de tuyau pour le transfert d'eau sous pression, d'air comprimé ou de vide.

13. Dispositif selon l'une des revendications 1 à 5, caractérisé par au moins un appareil d'essai rotatif avec une tête de sonde et un organe de liaison allongé, rigide, par un entraînement, placé dans l'unité de base, y compris un moteur (4), la pièce de liaison (9) rigide étant conçue pour la liaison amovible avec l'entraînement.

14. Dispositif selon la revendication 13, caractérisé en ce que l'appareil d'essai rotatif comporte une barre de support (28), pouvant être raccordée à l'entraînement, qui présente à une extrémité deux têtes de sonde (111, 112) diamétralement opposées, portées par une pièce transversale (110).

15. Dispositif selon la revendication 14, caractérisé en ce que les têtes de sonde (111, 112) sont en forme de demi-sphère ou en forme de goutte et sont reliées rigidement avec la pièce transversale (110), en ce que la pièce transversale (110) est un organe à tige allongée, qui peut être relié, dans différentes positions de rotation, avec la barre de support (28), de manière à pouvoir tourner avec les têtes de sonde (111, 112) autour de son axe longitudinal, de manière que la position de rotation des têtes de sonde autour de l'axe longitudinal de l'organe à tige puisse être choisie librement.
